(19)

(11) **EP 4 487 784 B1**

(12) **EUROPÄISCHE PATENTSCHRIFT**

(45) Veröffentlichungstag und Bekanntmachung des Hinweises auf die Patenterteilung:
**25.02.2026 Patentblatt 2026/09**

(21) Anmeldenummer: **23183627.1**

(22) Anmeldetag: **05.07.2023**

(51) Internationale Patentklassifikation (IPC):
***A61B 8/06*** $^{(2006.01)}$ ***A61B 8/08*** $^{(2006.01)}$
***A61B 8/00*** $^{(2006.01)}$ *A61B 5/00* $^{(2006.01)}$

(52) Gemeinsame Patentklassifikation (CPC):
**A61B 8/06; A61B 8/0891; A61B 8/4227; A61B 8/4483; A61B 8/488; A61B 8/5223**

(54) **MESSUNG EINES BLUTSTROMES IN EINEM BLUTGEFÄSS**

ULTRASONIC BLOOD FLOW MEASUREMENT FOR BLOOD VESSEL

MESURER UN FLUX SANGUIN DANS UN VAISSEAU SANGUIN PAR ULTRASONS

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(43) Veröffentlichungstag der Anmeldung:
**08.01.2025 Patentblatt 2025/02**

(73) Patentinhaber: **GAMPT mbH, Gesellschaft für Angewandte Medizinische Physik und Technik**
**06217 Merseburg (DE)**

(72) Erfinder:
- **FUCHS, Reinhard**
  **04107 Leipzig (DE)**
- **Dr. NEUMUTH, Thomas**
  **04277 Leipzig (DE)**
- **EGER, Andreas**
  **04229 Leipzig (DE)**
- **Dr. LENK, Karsten**
  **04275 Leipzig (DE)**
- **Dr. SCHULTZ, Michael**
  **06231 Bad Dürrenberg (DE)**
- **KLAUA, Robert**
  **06110 Halle (Saale) (DE)**
- **DIETRICH, Georg**
  **06198 Salzatal OT Zappendorf (DE)**
- **PAWLAK, Matthias**
  **06114 Halle (Saale) (DE)**

(74) Vertreter: **Maikowski & Ninnemann Patentanwälte Partnerschaft mbB**
**Postfach 15 09 20**
**10671 Berlin (DE)**

(56) Entgegenhaltungen:
**EP-A1- 3 505 071**
**US-A1- 2006 241 460**
**US-A1- 2017 332 995**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Beschrieben wird eine Ultraschallvorrichtung zur Messung von Fluidströmen, insbesondere zur Messung des Blutstromes in einem Blutgefäß. Die Ultraschallvorrichtung weist eine Messelektronik, mindestens drei Piezokeramiken und ein Gehäuse auf. Jede Piezokeramik ist erfindungsgemäß in einem anderen Winkel zur Schallaustrittsfläche des Gehäuses im Gehäuse angeordnet. Weiterhin weist das Gehäuse mindestens ein Mittel zum Befestigen des Gehäuses an einem Patienten oder einem Hilfsmittel auf.

Der arterielle Blutfluss durch die Halsschlagader (lat. Arteria Carotis Communis) kann als repräsentative Größe für die Einschätzung der Effektivität von Wiederbelebungsmaßnahmen dienen. Stand der Technik bei der Bewertung von Herzdruckmassagen sind nach wie vor Eindruck-Frequenz und Eindruck-Tiefe. Der tatsächlich verursachte Blutfluss und die damit verbundene Sauerstoffversorgung im Hirn sind dabei weder dem Helfer noch dem medizinischen Personal bekannt. In Tierversuchen konnte jedoch bereits gezeigt werden, dass das Überleben eines Herzstillstandes direkt abhängig vom zerebralen Blutfluss während der Wiederbelebungsmaßnahme ist [1]. So überleben nicht selten Patienten das Reanimationsereignis, können aber aufgrund einer infausten neurologischen Prognose dennoch nicht genesen. Auch wenn es gelingt einen Spontankreislauf zu erlangen, ist das neurologische Outcome der Patienten direkt abhängig von der Qualität der Reanimation und der Minimierung der "No-Flow"-Zeiträume, in denen kein sauerstoffreiches Blut ins Gehirn transportiert wird.

Die neuronalen Strukturen des Hirns reagieren sensibel auf eine Sauerstoffunterversorgung und nehmen frühzeitig schwere, irreversible Schäden [2]. Die Folgen einer ineffektiven Widerbelebung sind unter Umständen erst im späteren Verlauf der Therapie bemerkbar. Der scheinbar stabile Zustand des Patienten kann unter diesen Umständen zu einer verfrühten Entlassung führen, bei dem ein Rückfall in den Herzstillstand erst nach dem Verlassen der Klinik erfolgt. Eine Erfassung des Volumenstroms innerhalb der Carotis, während der Widerbelebungsmaßnahme, kann den Helfer in seiner Leistung maßgeblich unterstützen, indem es bei einer Unterversorgung ein Signal auslöst. Es bedarf somit der Messung dieses aussagekräftigen Surrogatparameters, welcher im außer- und innerklinischen Szenario Aufschluss über die Qualität der Herzdruckmassagen geben kann und einen indirekten Rückschluss auf die Blutversorgung des Gehirns erlaubt.

Zur Ermittlung des zeitlich aufgelösten Blutflusses in Arterien und Venen werden innerklinisch zwei Methoden am häufigsten eingesetzt. Bei Patienten mit invasiven arteriellen Zugängen, kann der Blutfluss während der Reanimation über Druckkurven erfasst und aufgezeichnet werden. Invasive Zugänge für die Blutdruckmessung sind außerhalb von Intensivstationen jedoch weniger vorzufinden und werden im ambulanten Einsatz normalerweise erst nach erfolgter Reanimation des Kreislaufs gelegt.

Eine schnelle und als generell nicht-invasiv angesehene Alternative bieten moderne Ultraschallgeräte, mit Doppler-Sonografie-Funktion mit dem B-Bild Modus kombiniert. Die Dopplersonographie nutzt durch den Doppler-Effekt entstehende gemessene Frequenzunterschiede zwischen ausgesendeten und empfangenen Ultraschallsignalen, zur Erfassung von sich bewegender Materie. Die von roten Blutkörperchen reflektierte Ultraschallwellen können somit genutzt werden, um die Geschwindigkeit des Blutstroms während der Reanimation zu erfassen.

Eine dopplersonographische Messung des arteriellen Blutflusses in der Carotis Communis ist während der Reanimation prinzipiell bereits möglich, Geräte für den stationären Betrieb sind für diese Anwendung jedoch nicht praktikabel. Die verwendeten Ultraschallköpfe und Computer für die Messung/Analyse sind zu groß und auf eine Energieversorgung durch den Netzbetrieb angewiesen. Zudem bedürfen die Ultraschallköpfe einer manuellen, händischen Bedienung, eine langfristige, feste Anbringung am Menschen, bei der niemand das Messgerät halten muss, ist effektiv nicht möglich.

Dem Gerät mangelt es jedoch auch an zusätzlichen Befestigungen und einer Kompaktheit, welche eine schnelle, einfach zu handhabende und robuste Anbringung des Ultraschallkopfs über einer Schlagader ermöglicht, ohne diese zu kompromittieren. Die Interpretation der Resultate von B-Bild und Dopplersonographie bedürfen zudem einer gewissen Expertise, die im Rettungsdienst nicht flächendeckend vorhanden ist. Hier wäre daher eine automatisierte Auswertung der hämodynamischen Kenngrößen erforderlich. Dies bedarf wiederum zusätzlicher Signalverarbeitungsschritte für die Kompensation von Artefakten, die durch Bewegungen des Körpers und der Ultraschallwandler entstehen.

Portable Geräte für die Ultraschallmessung sind bereits käuflich und besitzen unter anderem auch die Möglichkeit der dopplersonographischen Messung. Der L7 Linear Handheld Ultrasound Scanner der Firma Clarius (Vancouver, CA) ist ein solches Gerät, welches über mehrere integrierte Ultraschallwandler eine Erfassung von Gewebestrukturen und aufkommender Bewegungen ermöglicht.

Eine weitere Lösung wurde von der Forschungsgruppe um Joseph Eibl et al. (EP 3823536) entwickelt. Hierbei handelt es sich um ein Ultraschallmodul, das an den Hals eines Patienten geklebt wird und daraufhin eine dopplersonografische Messung durchführt. Die Frequenzunterschiede werden im Modul erfasst und ausgewertet, kabellose Kommunikation ermöglicht die Visualisierung der Messungen auf externen Geräten, dies können Tablets oder ähnliche Computergeräte sein. Das Ultraschall-Pflaster ist für eine längerfristige Erfassung und Auswertung des Blutflusses in menschlichen Gefäßen gedacht. Die Messung erfolgt mithilfe kontinuierlicher Doppler-Messung, sie findet über zwei Piezo-Keramiken innerhalb des Gerätes statt, von denen eine ununterbrochen Ultraschallsignale sendet deren Reflektionen die andere Keramik kontinuierlich empfängt.

Handhabung, Anbringung und Messverfahren offenbaren jedoch einen Verbesserungsbedarf. Die LEDs des Geräts sind nur für die Anzeige des Status der Bluetooth-Verbindung, es mangelt dem System daher an einer autarken Möglichkeit dem Nutzer über die Qualität des erzeugten Blutflusses zu informieren. Gemessene Werte müssen über ein zusätzliches Gerät angezeigt werden, ein Umstand, der die Nutzung für den Anwender in der Notfallsituation weniger attraktiv gestaltet. So wird der Vorteil der Befestigung des Gerätes am Patienten aufgehoben, indem nun ein Tablet oder PC für eine kurze Zeit gehandhabt werden muss, um eine Anzeigemöglichkeit vorzubereiten. Die Anbringung über den Klebestreifen ermöglicht zwar die kontinuierliche Messung ohne zusätzliche Handhabung des Gerätes, allerdings erlaubt es effektiv keine schnelle Anpassung der Position, welche bei einer ungenauen Anbringung des Pflasters oberhalb der Carotis oder einer Verschiebung des Gefäßes die Effektivität der Messung erheblich beeinflussen würde. Die Methode der Messung, welche über Continuos-Wave-Doppler mit einem Sender und einem Empfänger erfolgt, besitzt den Vorteil einer großen Abdeckung der durchschallten Fläche, allerdings werden dadurch auch ein Großteil an Störsignalen (z.B. Gewebebewegungen) erfasst, deren Filterung sich auf die Qualität des Blutflusssignals auswirken kann. Dieser Nachteil verschärft sich durch die während der Reanimation ausgeübten Bewegungsartefakte, wie in den Farbdoppler Aufnahmen während einer Reanimation von Koch et. al. zu sehen ist [3]. Zudem ist die quantitative Positions- und Lageerfassung der Carotis unter Verwendung nur einer Empfangssonde nicht praktikabel, sodass eine genaue Bestimmung des quantitativen Blutflusses schwer realisierbar ist. Sofern während der Reanimationstätigkeiten die Fläche oberhalb der Carotis freigelegt werden muss, ist zudem eine schnelle Abnahme und erneute Anbringung des Systems nicht möglich. Aufgrund der Verwendung von nur einem Empfangselement ist zudem eine Winkelbestimmung zwischen Gefäßachse und Schallbündel nicht möglich. Individuelle Lagen der Karotis gegenüber der Hautoberfläche können in der Berechnung nicht berücksichtigt werden, wodurch die Blutflussberechnung verfälscht wird.

Die beschriebenen Lösungen ermöglichen entweder keine längerfristige Messung, ohne dass ein Anwender die Ultraschallköpfe in Position halten muss, oder erlauben nach der Anbringung des Ultraschallmoduls am Hals keine Veränderung der Messstelle.

Die geringe Flexibilität in der Anbringung des gewünschten Systems birgt einen großen Nachteil, da während der Reanimation keine Korrekturen bezüglich der Messposition vorgenommen werden können. Zudem kann die belegte Fläche am Hals nicht schnell von der Messtechnik befreit werden, um sie nach erfolgten Umlagerungen, Legung invasiver Zugänge oder anderen Tätigkeiten wieder schnell und einfach anbringen zu können. Darüber hinaus sind die Geräte auf externe Systeme angewiesen und ermöglichen keine direkte Information an den Nutzer.

Die WO 2017/096487 offenbart eine tragbare Überwachungsvorrichtung, die ausschließlich mit Wandlerpaaren arbeitet.

EP 3 505 071 A1 offenbart eine Ultraschallkopf für eine continuous-wave Ultraschallvorrichtung zur Messung eines Blutstroms am Patienten.

US 2006/241460 A1 offenbart eine Vorrichtung zur Rheologiemessung an Blut und ein zugehöriges Verfahren unter Nutzung von Ultraschall.

US 2017/332995 A1 offenbart ein System und ein Verfahren zur automatisierten Beurteilung von hämodynamischen Eigenschaften eines Patienten.

Ausgehend vom Stand der Technik ist es daher die Aufgabe der vorliegenden Erfindung die Nachteile aus dem Stand der Technik zu beheben und ein Verfahren zur Messung eines Blutstromes in einem Blutgefäß zur Verfügung zu stellen, welches zuverlässig direkt eine quantitative Information über den Blutfluss in einem Blutgefäß gibt, wobei die verwendete Ultraschallvorrichtung schnell angebracht und wieder entfernt werden kann.

Hierfür stellt die Erfindung ein Verfahren gemäß den Ansprüchen 1 bis 3 bereit.

## Detaillierte Beschreibung

Die Ultraschallvorrichtung dient der Messung eines Blutstromes in einem Blutgefäß. Die Ultraschallvorrichtung weist eine Messelektronik, mindestens drei Piezokeramiken und ein Gehäuse auf. Es sind die mindestens drei Piezokeramiken im Gehäuse angeordnet.

In einer Ausführung weist das Gehäuse genau drei Piezokeramiken auf.

Jede Piezokeramik ist im Gehäuse in einem anderen Winkel zur Schallaustrittsfläche des Gehäuses angeordnet. Hierfür wird der Winkel der Normalen der Piezokeramik zur Normalen der Schallaustrittsfläche betrachtet. Dabei wird die Gehäusewand, durch die die von den Piezokeramiken erzeugten Schallwellen Richtung Körpergewebe austreten und durch die die reflektierten Ultraschallwellen in das Gehäuse hineingelangen als Schallaustrittsfläche bezeichnet.

In einer Ausführung können die Piezokeramiken in einem Winkel zwischen 90° und 0°, bevorzugt zwischen 75° und 0° zur Schallaustrittsfläche des Gehäuses angeordnet sein. Die Winkel der Piezokeramiken unterscheiden sich dabei zwischen $\pm 5°$ und $\pm 30°$, bevorzugt zwischen $\pm 10°$ und $\pm 25°$.

Die Piezokeramiken sind im Gehäuse auf einer Koppelstrecke aufgebracht. Die Koppelstrecke besteht bevorzugt aus biokompatiblen Materialien mit geringer Dämpfung und guter Impedanzanpassung an die Piezokeramiken und die Haut. Durch die Konstruktion des Gehäuses sind die Positionen und damit die Winkel der Piezokeramiken im Gehäuse bekannt.

Als Piezokeramik kann jede herkömmliche, im Ultraschallbereich anwendbare Keramik verwendet werden. Vorzugsweise wird eine Kompositkeramik verwendet. Besonders bevorzugt ist die Verwendung einer gefüllten Piezokeramik, wie beispielsweise einer Blei-Zirkonat-Titanat-Keramik (PZT-Keramik) mit Polymerfüllung. Die Geometrie der Piezokeramik ist so gewählt, dass ein im Wesentlichen homogenes Schallfeld erzeugt wird. Die Piezokeramik hat vorzugsweise einen rechteckigen oder elliptischen Querschnitt. Die Größe der Piezokeramiken ist dabei so gewählt, dass diese im Gehäuse Platz finden und die Schalaustrittsfläche des Gehäuses am Hals eines Patienten vollflächig angelegt werden kann.

Alle Piezokeramiken sind dazu eingerichtet continous wave-Doppler Ultraschallsignale (cw) und/oder gepulste Doppler Ultraschallsignale (burst) auszusenden.

Die Vorrichtung ist dazu eingerichtet in mindestens drei Modi zu arbeiten. Wobei ein Modus einen bestimmten Messablauf bezeichnet. In verschiedenen Modi sind beispielsweise unterschiedliche Piezokeramiken für die Aussendung und/oder den Empfang von Ultraschallsignalen vorgesehen.

Der erste Modus, der im Folgenden mit Modus 0 bezeichnet wird, diente der Einschätzung der korrekten Lage und Ankopplung der erfindungsgemäßen Ultraschallvorrichtung an einem Patienten. Im Modus 0 arbeitet eine Piezokeramik als Sender während alle verfügbaren Piezokeramiken als Empfänger arbeiten. Werden Puls-Dopplersignale gesendet, arbeiten alle Piezokeramiken, auch die Sendkeramik als Empfänger.

Ein zweiter Modus, im Folgenden mit Modus 1 bezeichnet, dient zur Ermittlung der Gefäßtiefe, des Gefäßdurchmessers und der Dopplerwinkel der Piezokeramiken. Im Modus 1 werden nacheinander alle Piezokeramiken als Sender verwendet, während alle verfügbaren Piezokeramiken das reflektierte Ultraschallsignal zeitgleich empfangen. Werden Puls-Dopplersignale gesendet, arbeiten alle Piezokeramiken, auch die Sendkeramik als Empfänger.

Ein dritter Modus, im Folgenden mit Modus 2 bezeichnet, dient der quantifizierten Blutflussmessung. In Modus 2 dient eine Piezokeramik als Sender während alle verfügbaren Piezokeramiken zeitgleich als Empfänger dienen. Werden Puls-Dopplersignale verwendet, arbeiten alle Piezokeramiken, auch die Sendkeramik als Empfänger. In einer bevorzugten Ausführungsform ist die Ultraschallvorrichtung dazu eingerichtet, in mindestens 3 Modi zu messen, wobei in allen Modi eine Piezokeramik als Sender arbeitet und alle verfügbaren Piezokeramiken als Empfänger arbeiten, wobei in mindestens zwei Modi alle verfügbaren Piezokeramiken, die als Empfänger arbeiten, zeitgleich als Empfänger arbeiten.

Zeitgleich bedeutet im Zusammenhang der vorliegenden Erfindung, dass die jeweiligen Piezokeramiken zur genau gleichen Zeit das an den Piezokeramiken empfangene Ultraschallsignal messen.

Durch die gleichzeitige Messung des Ultraschallsignals unter mindestens zwei verschiedenen Winkeln wird eine sehr genaue Bestimmung des Dopplerwinkels möglich. Der Dopplerwinkel bezeichnet den Winkel zwischen Schallweg und Flussrichtung des Blutstroms im Blutgefäß. Die Winkel der Piezokeramiken zur Schallaustrittsfläche sind aus der Konstruktion der Ultraschallvorrichtung bekannt.

Zur Steuerung der Piezokeramiken weist die Ultraschallvorrichtung weiterhin eine entsprechende Messelektronik auf. Die Messelektronik ist für die Energieregelung, Messwerterfassung, Demodulation, Vertonung und Auswertung der Dopplersignale verantwortlich. Die Messelektronik leitet unter anderem elektrische Energie zur Ultraschallerzeugung an die Piezokeramiken weiter. Weiterhin weist das Gehäuse eine Platine auf, von der eine mehrpolige Kabelverbindung zur Messelektronik geht. Weiterhin ist die Platine mit den Piezokeramiken verbunden und für die Verstärkung und Demodulation von gesendeten und gemessenen Signalen verantwortlich.

In einer Ausführung sind die Messelektronik und die Platine dazu eingerichtet für jede Piezokeramik separate aber auch aufeinander synchronisierte Sende- und Empfangsperioden zu aktivieren.

In einer Ausführung weist die Messelektronik weiterhin einen Datenspeicher auf. Auf den Datenspeicher können die Messsignale der Ultraschallvorrichtung gespeichert werden. Dies ermöglicht es die Messdaten während eines Animationsvorganges im Nachhinein auszuwerten und beispielswiese in einer elektronischen Patientenakte zu hinterlegen.

In einer Ausführung weist die Messelektronik daher weiterhin einen Ausgang für eine Verbindung zu einem Netzwerk, beispielsweise über WLAN, Bluetooth oder Kabel, und/oder einen Ausgang für ein externes Speichermedium beispielsweise einen USB-Anschluss auf. Über diesen Ausgang bzw. diese Ausgänge können gespeicherte Daten ausgelesen werden.

In einer Ausführung der vorliegenden Erfindung ist die Messelektronik im gleichen Gehäuse wie die mindestens drei Piezokeramiken angeordnet. In einer weiteren Ausführung ist die Messelektronik in einem weiteren Gehäuse beabstandet von dem Gehäuse mit den mindestens drei Piezokeramiken angeordnet. In dieser Ausführung weist die Ultraschallvorrichtung entsprechend zwei Gehäuse auf. Die Gehäuse sind durch geeignet Kabel miteinander verbunden.

Die Energieversorgung der Ultraschallvorrichtung erfolgt bevorzugt über einen geeigneten portablen Energiespeicher und/oder über eine Verbindung zum Stromnetz über einen Adapter.

Der durch die Ultraschallvorrichtung ausgesendete Ultraschall liegt im Bereich des nichtinvasiven diagnostischen Ultraschalls. Nichtinvasiver diagnostischer Ultraschall wird in der DIN EN 60601-2-37 (Medizinische elektrische Geräte Teil 2-37) definiert. Nicht invasiver Ultraschall stellt definitionsgemäß keinen Eingriff am untersuchten Körper dar.

In einer Ausführung weist das Gehäuse weiterhin mindestens ein Mittel zum Befestigen des Gehäuses an einem Patienten oder an einem Hilfsmittel auf. Bevorzugt wird die Ultraschallvorrichtung durch das mindestens eine Mittel zum Befestigen des Gehäuses am Hals eines Patienten angebracht. Die Ultraschallvorrichtung wird dabei lösbar und nichtinvasiv am Körper angebracht. Wird die Ultraschallvorrichtung durch das mindestens eine Mittel zum Befestigen an einem Hilfsmittel angebracht, so ist das Hilfsmittel wiederum bevorzugt dazu geeignet, am Hals eines Patienten angebracht zu werden. In einer Ausführung ist das Hilfsmittel, an dem das Gehäuse mit dem mindesten einen Mittel zur Befestigung angebracht werden kann, eine Laerdal Stiffneck Halskrause.

In einer bevorzugten Ausführung weist das Gehäuse genau ein Mittel zum Befestigen des Gehäuses an einem Patienten oder an einem Hilfsmittel auf.

Das mindestens eine Mittel zum Befestigen ist dabei ausgesucht aus der Gruppe aufweisend Halterungen aus Kunststoff, Halskrausen und Manschetten, insbesondere Halskrausen und Manschetten aus dehnbarem Textil. Durch das mindestens eine Mittel zum Befestigen kann die Ultraschallvorrichtung am Körper, insbesondere am Hals eines Menschen fixiert werden.

Aus dem Stand der Technik sind Vorrichtungen bekannt, die mit selbstklebenden Materialien am Hals fixiert werden. Nachteilig hieran ist unter anderem, dass Behaarung am menschlichen Körper das Anbringen und den Hautkontakt erschweren oder sogar unmöglich machen kann. Darüber hinaus ist der Zugang zum Hals durch das Kleben beispielsweise in Form eines Pflasters blockiert. Nach dem Entfernen der selbstklebenden Materialien können zudem Hautirritationen auftreten oder vorhandene Hautkomplikationen verschlimmert werden. Das Anbringen durch ein selbstklebendes Material zeigt sich zudem als weniger robust gegenüber Halsrotationen oder Gewebebewegungen, da der Hals nicht fixiert ist. Dadurch verschiebt sich auch die Region in der gemessen wird und unter Umständen wird dann ein Blutgefäß nicht mehr ausreichend abgedeckt.

Die Vorrichtung verzichtet auf selbstklebende Materialien zur Befestigung der Ultraschallvorrichtung am Körper. Besonders vorteilhaft ist dies, wenn der Blutfluss in einem Blutgefäß am Hals eines Patienten gemessen werden soll. Ein zentraler Punkt bei der Messung des Blutflusses in einem Blutgefäß ist die korrekte Positionierung der messenden Piezokeramiken über dem betreffenden Blutgefäß. Hier bietet die Ultraschallvorrichtung den Vorteil, dass die Position der Ultraschallvorrichtung wenn nötig schnell verändert und angepasst werden. Hautirritationen werden vermieden und auch bei einer Behaarung des Patienten ist ein Anbringen am Hals unproblematisch möglich.

In einer Ausführung ist das Gehäuse mit den mindestens drei Piezokeramiken durch eine Halterung aus Kunststoff an einer Laerdal-Stiffneck Halskrause anbringbar. Das Gehäuse ist dabei so angeordnet, dass die Schallaustrittsfläche am Hals eines Patienten anliegt. In einer Ausführung ist die Messelektronik nicht im Gehäuse der mindestens drei Piezokeramiken angeordnet, sondern in einem weiteren Gehäuse. Dieses weitere Gehäuse kann an der vom Patienten abgewandten Seite der Laerdal-Stiffneck Halskrause angebracht sein.

In einer weiteren Ausführung, kann mithilfe von manuell aufblasbaren Luftkissen die Position und der Anpressdruck am Hals je nach Patient angepasst werden. Eine Betätigung eines Rückschlagventils einer Pumpe und die Entriegelung eines Schlussmechanismus der Halskrause garantieren eine unkomplizierte Abnahme der Ultraschallvorrichtung. In einer Ausführung weist das Gehäuse der Ultraschallvorrichtung daher weiterhin mindestens ein aufblasbares Luftkissen auf.

In einer Ausführung wird die Position der Ultraschallvorrichtung durch Verschieben der Halterung an der Halskrause geändert, wobei aufgrund des Anpressdrucks die Position der Ultraschallvorrichtung bei geschlossener Halskrause nicht veränderlich ist.

In einer Ausführungsform wird das Gehäuse mit den mindestens drei Piezokeramiken über ein dehnbares Textil, ein Gummiband oder eine eigens entwickelte Halskrause am Hals eines Patienten fixiert. Das Gummiband kann hierbei beispielsweise durch das Gehäuse hindurchgeführt werden und zwischen der Messelektronik und den mindestens drei Piezokeramiken und der Koppelstrecke verlaufen. Dabei wird die Ultraschallvorrichtung wiederum so am Hals angebracht, dass die Schallaustrittsfläche am Hals des Patienten anliegt.

In einer Ausführungsform werden die gemessenen Signale auf einen externen Bildschirm übertragen. Hierfür weist die Ultraschallvorrichtung entsprechende Anschlüsse zur Datenübertragung (WLAN, Bluetooth, Kabel o.ä.) auf.

In einer weiteren Ausführung weist die Ultraschallvorrichtung weiterhin mindestens einen Signalgeber in Form einer Anzeige und/oder einer akustischen Signalausgabe auf. Die Vorrichtung verzichtet damit auf die Anbindungen von Drittgeräten, Monitore o.ä.. Anstatt der Datenübertragung per Bluetooth oder Wireless-LAN werden Messwerte sofort durch die Messelektronik ausgewertet und für die Anzeige genutzt. Die Anzeige und/oder das akustische Signal geben damit unmittelbar einen Rückschluss über den Blutstrom in dem überwachten Blutgefäß. Zu diesem Zweck werden in einer Ausführungsform LEDs genutzt, die den approximierten, momentanen Blutflusswert anzeigen und/oder die Signalstärke des Messsignals und/oder die Bewertung des approximierten Blutflusses während der vergangenen Minute(n).

In einer Ausführungsform befinden sich der Signalgeber am Gehäuse der Ultraschallvorrichtung und ist derart angeordnet, dass der Signalgeber gut zu sehen ist.

Ist die Messelektronik in einem weiteren Gehäuse angeordnet, welches vom Gehäuse mit den mindestens drei Piezokeramiken beabstandet ist, so kann in einer Ausführungsform der Signalgeber auch an dem Gehäuse angeordnet sein, welches die Messelektronik umfasst.

In einer bevorzugten Ausführungsform kann so der Blutfluss in einem Blutgefäß beispielsweise während der Reanimation eines Patienten gemessen und dargestellt werden.

Weist die Ultraschallvorrichtung mindestens einen Signalgeber auf, so wird der Signalgeber durch die Messelektronik im Gehäuse gesteuert.

Weiterhin umfasst die Erfindung ein nichtinvasives Verfahren zur Messung des Blutstromes in einem Blutgefäß mit einer Ultraschallvorrichtung, aufweisend die Schritte

a) Ausführen einer Messung mit einem Modus 0, in dem eine Piezokeramik als Sender arbeitet, während alle verfügbaren Piezokeramiken als Empfänger arbeiten;
b) Beurteilung ob bei der Messung mit dem Modus 0 ein blutdurchströmtes Blutgefäß erfasst wird anhand der Messwerte mit dem Modus 0 und Ausgabe einer Information, ob bei der Messung ein blutdurchströmtes Blutgefäß erfasst wird;
c) Ausführen einer Messung mit einem Modus 1, indem nacheinander jede Piezokeramik als Sender arbeitet, während alle verfügbaren Piezokeramiken als Empfänger arbeiten, wobei die reflektierten Ultraschallsignale von den Empfängern zeitgleich aufgenommen werden und wobei für jede Piezokeramik die Messtortiefe (Laufzeitfenster) variiert wird;
d) Berechnung der Gefäßtiefe, des Gefäßdurchmessers und der Dopplerwinkel der einzelnen Piezokeramiken aus den Messdaten der Messung im Modus 1;
e) Ausführen einer Messung mit einem Modus 2, indem eine Piezokeramik als Sender arbeitet, während alle verfügbaren Piezokeramiken als Empfänger arbeiten, wobei die reflektierten Ultraschallsignale von den Empfängern zeitgleich aufgenommen werden;
f) Berechnung der Geschwindigkeit des Blutflusses aus den Messsignalen der Messung mit Modus 2 und der berechneten Gefäßtiefe, des Gefäßdurchmessers und der Dopplerwinkel der Piezokeramiken.

Alle Merkmale, die für die Ultraschallvorrichtung beschrieben sind, gelten in gleicher Weise für das erfindungsgemäße Verfahren und umgekehrt.

In einer Ausführungsform der vorliegenden Erfindung wird die Ultraschallvorrichtung über eine Halterung auf der Haut am Hals eines Patienten angebracht.

Grundlegend kann durch das erfindungsgemäße Verfahren ein Reflektionssignal durch die Gefäßwand und ein Reflektionssignal durch die Streuung an den Erythrozyten erhalten werden. Hierdurch können entsprechend Wandbewegungen und Bewegungen der Erythrozyten detektiert werden. Die Bewegung der Erythrozyten ebenso wie die Wandbewegung wird durch den Blutfluss verursacht.

Nach erfolgter Erzeugung des Ultraschallsignals wird die Energiezufuhr bei Nutzung von gepulsten Doppler-Ultraschallsignalen unterbrochen, um die Reflektionen der ausgesendeten Ultraschallwellen empfangen zu können. Je nach Geschwindigkeit der roten Blutkörperchen und des Winkels der gesendeten Ultraschallwellen zur Gefäßachse erfahren die reflektierten mechanischen Wellen eine Frequenzverschiebung durch den Dopplereffekt.

In einem Verfahrensschritt wird zunächst eine Messung mit einem Modus 0 ausgeführt, in dem eine Piezokeramik als Sender arbeitet, während alle verfügbaren Piezokeramiken als Empfänger arbeiten. Alle verfügbaren Piezokeramiken arbeiten als Empfänger bedeutet, dass im cw-Betrieb eine Piezokeramik sendet während alle übrigen Piezokeramiken empfangen und im Puls-Doppler Betrieb alle Piezokeramiken inklusive der Piezokeramik, die als Sender agiert, empfangen. Die Synchronisierung der Messung findet über systeminterne Trigger statt.

Der Modus dient der Einschätzung der korrekten Lage und Ankopplung des Wandlers an das zu vermessende System (z.B. Hals).

In einer bevorzugten Ausführungsform wird im Modus 0 die Piezokeramik als Sender verwendet, deren Winkel zur Schallaustrittsfläche sich am geringfügigsten von 90° unterscheidet. Ist eine Piezokeramik genau im Winkel von 90° zur Schallaustrittsfläche in der Ultraschallvorrichtung angeordnet, so wird die Piezokeramik verwendet, deren Winkel zur Schallaustrittsfläche sich am wenigsten von der Piezokeramik mit senkrechtem Winkel zur Ultraschallaustrittsfläche unterscheidet. Damit ergibt sich ein guter Kompromiss für das Streusignal der Blutkörperchen (möglichst großer Dopplerwinkel) und das Reflektionssignal der Wandbewegung (möglichst kleiner Dopplerwinkel). Die Tortiefen für die Puls-Doppler-Messung wird bevorzugt gering gewählt und die Torgröße hoch. Die Wahl geeigneter Parameter ist dem Fachmann vertraut. Die Tortiefe ist gleichbedeutend mit der Messtortiefe, die auch als Laufzeitfenster bezeichnet wird.

In einer Ausführungsform werden für die schnelle Anpassung des Tores zusätzlich Parameterprofile für verschiedene Halsproportionen eines Patienten bereitgestellt, aus denen der Nutzer eines auswählt, welches den Halsproportionen des Patienten entspricht. Beispielsweise soll bei einer Messung an einem durchschnittlich proportionierten Hals mit geringem Fettanteil ein Messbereich zwischen 12 mm und 30 mm Tiefe im Körpergewebe des Patienten abgedeckt werden. Bei einer Schallgeschwindigkeit von 1,5 mm/µs ergibt sich eine Tortiefe/Empfangszeit (Hin- und Rückweg bedeutet Verdopplung) von

$$\frac{12mm}{1{,}5mm/\mu s} * 2 = 8\mu s * 2 = 16\mu s$$

und eine Torgröße/Empfangsdauer (halbe Tortiefe subtrahieren) von

$$\frac{30mm}{1{,}5mm/\mu s} * 2 - 8\mu s = 24\mu s$$

Die von den jeweiligen Piezokeramiken gemessenen Reflektionen werden für die Berechnung von Spektrogrammen genutzt. Diese können mit Leistung-/Power-/Intensität-Grenzwerten und Wertebereichen angepasst werden. Anhand der niederfrequenten Leistungen im Spektrogramm kann eine visuell-manuelle oder automatische Bestätigung erfolgen, ob das eingestellte Doppler-Tor ein blutdurchströmtes Gefäß abdeckt. Bei erfolgreicher Abdeckung enthalten die Spektrogramme periodisch hohe Signalleistungen/-intensitäten/-Power in den niedrigen Frequenzen, die durch Gefäßwandbewegungen entstehen. Für eine automatische Beurteilung werden hinterlegte Referenzwerte genutzt, mit denen die Messsignale verglichen werden. Erfindungsgemäß findet daher ein Vergleich der Messwerte mit Referenzwerten und eine Beurteilung, ob bei der Messung ein blutdurchströmtes Blutgefäß erfasst wird, statt. Anschließend erfolgt eine Ausgabe einer Information darüber, ob bei der Messung ein blutdurchströmtes Blutgefäß erfasst wird. Die Ausgabe der Information kann auf einem angeschlossenen Bildschirm und/oder durch LED's und/oder die Ausgabe eines akustischen Signals erfolgen.

In einer Ausführungsform der vorliegenden Erfindung kann die Position der Ultraschallvorrichtung am Patienten verändert werden, falls kein blutdurchströmtes Blutgefäß in der Messung erfasst wird.

Verfahrensgemäß wird weiterhin eine Messung mit einem Modus 1 ausgeführt. Im Modus 1 arbeitet nacheinander jede Piezokeramik als Sender, während alle verfügbaren Piezokeramiken als Empfänger arbeiten. Mit jeder Piezokeramik werden dabei Messungen mit unterschiedlichen Tortiefen durchgeführt. Die reflektierten Ultraschallsignale werden von den Empfängern dabei zeitgleich aufgenommen.

Der Modus dient zur Ermittlung der Gefäßtiefe, des Gefäßdurchmessers und des Dopplerwinkels jeder Piezokeramik in der erfindungsgemäßen Ultraschallvorrichtung.

Im Modus 1 werden erfindungsgemäß alle Piezokeramiken nacheinander als Sender verwendet. Während eine Piezokeramik sendet, empfangen alle zur Verfügung stehenden Piezokeramiken die Reflektionen und Streusignale. Das alle verfügbaren Piezokeramiken als Empfänger arbeiten, bedeutet, dass im cw-Betrieb eine Piezokeramik sendet während alle übrigen Piezokeramiken empfangen und im Puls-Doppler Betrieb alle Piezokeramiken inklusive der Piezokeramik, die als Sender agiert, empfangen. Die Synchronisierung der Messung findet über systeminterne Trigger statt.

Die Messung erfolgt in mehreren Messschleifen. Die Tortiefe der Messung wird für alle empfangenden Piezokeramiken iterativ erhöht. Bei jedem Schritt der Torverschiebung wird für eine definierte Zeit gemessen. In einer Ausführungsform ist die Torgröße für jeden Tortiefe gleich. In dieser Ausführungsform umfasst eine Messschleife eine Iterationsschleifen über die Tortiefe.

In einer weiteren Ausführungsform kann für bei jeder Tortiefe zusätzlich die Torgröße iterativ verändert werden. In dieser Ausführungsform umfasst eine Messschleife zwei Iterationsschleifen durchgeführt, eine über die Torgröße und eine über die Tortiefe.

Eine Messung mit dem Modus 1 bedeutet, dass Messchleifen für alle Piezokeramiken der Ultraschallvorrichtung durchlaufen werden.

Wenn alle Messungen für die sendende Piezokeramik abgeschlossen werden, wird ihre Sendefunktion eingestellt und eine andere Piezokeramik kann die Funktion des Senders übernehmen. Daraufhin kann die Messschleife erneut durchgeführt werden.

Jede Messung innerhalb einer Messschleife besteht aus der Erfassung der Doppler-Daten für jede Piezokeramik und der Berechnung des Spektrogramms, welches die Verteilung der Leistung (engl. Power) entlang des Frequenzbereichs darstellt. Die empfangenen Signalverläufe werden von ihrer Trägerfrequenz demoduliert und auf ihre spektralen Eigenschaften mithilfe einer Short-Time Fourier Transformation untersucht, deren Resultat, das Verhältnis der Signalleistung in Abhängigkeit der zugehörigen Frequenzen, angereiht entlang der Zeit das Spektrogramm ergibt. Durch eine Grenzwertanwendung innerhalb des Spektrogramms können über eine einhüllende Funktion die maximalen Frequenzen mit hinreichender Leistung ermittelt werden, die die Piezokeramiken empfangen haben.

Für jede Messung kann überprüft werden, ob eine Erfassung eines Blutgefäßes mit der Toreinstellung der jeweiligen Piezokeramik wahrscheinlich ist, solange dieses von Blut durchflossen wird. Die Doppler-Daten werden in einer Ausführungsform für jede Toreinstellung für jeden Wandler gemessen und gespeichert. Bevorzugt findet eine Unterscheidung zwischen niedrig- und höher-frequenten Bereichen der errechneten Spektrogramme und somit eine Trennung zwischen Gefäßwandbewegungen und Blutstreusignalen, über vordefinierte Schwellwerte statt. Diese Schwellwerte sind in der Messelektronik hinterlegt.

Eine Piezokeramik, die einen senkrechten Winkel zur Gefäßachse besitzt, besitzt keine Leistungen in den höher-frequenten Bereichen, die den Blutfluss widerspiegeln, aber in den geringen Doppler-Frequenzen, die die Wandbewegungen widerspiegeln. Bei einem Vergleich zwischen den verschiedenen Spektrogrammen der Wandler kann so die Piezokeramik mit dem geringsten Winkelunterschied zu 90° zur Schallaustrittsfläche ermittelt werden, wenn mehrere Wandler entlang der Torverschiebung unterschiedliche Signalintensitäten oberhalb des Frequenz-Schwellwertes besitzen. Ein Vergleich der nieder-frequenten Signalleistungen dieser Piezokeramik entlang der unterschiedlichen Tortiefen wird zu einer Bestimmung der Gefäßtiefe und einer Approximation des Gefäßdurchmessers genutzt.

Treten in den Spektrogrammen einer Piezokeramik Gefäßwandbewegungen auf, deren Leistungen proportional mit der Tortiefe zunehmen bzw. abnehmen, kann für diese Piezokeramik der Abstand zum Gefäß bestimmt werden.

In einer Ausführungsform erfolgt die Berechnung einer einhüllenden Funktion der maximalen Intensität entlang der Zeitachse des Spektrogramms. Wenn die einhüllende Funktion Anzeichen periodischen Verhaltens im gewünschten Frequenzbereich des Pulses eines Menschen oder des Wiederbelebungstakts (54 BPM - 120 BPM) besitzt, kann dies als Indiz genommen werden, dass die zugehörige Piezokeramik mit hoher Wahrscheinlichkeit den Blutfluss im Gefäß erfasst.

Decken mehrere Wandler das blutdurchflossene Gefäß mit ihrem Schallfeld ab, kann aufgrund der starren Winkelbeziehungen zwischen den Wandlern die Gefäßlage und damit der exakte Dopplerwinkel berechnet werden. Spektrogramm Daten werden dann durch Substitutionen der Dopplerwinkel von Frequenzwerten zu quantifizierten Geschwindigkeitswerten umgerechnet.

Erfindungsgemäß wird durch einen Vergleich der Signalleistungen der einhüllenden Funktionen im Blutfluss-Frequenzbereich (aller Spektrogramme aus allen Doppler-Konfigurationen) die beste Parametrisierung bzgl. Sender-Empfänger-Einstellung und Toreigenschaften ermittelt. Das heißt es wird die optimale Piezokeramik als Sender ermittelt und die zugehörige optimale Tortiefe und gegebenenfalls Torlänge.

Der Parametersatz und alle erfassten Doppler-Daten sowie die daraus ermittelten Parameter werden bevorzugt in der Messelektronik abgespeichert.

Aus den Messdaten der Messung im Modus 1 werden erfindungsgemäß die Gefäßtiefe, der Gefäßdurchmesser und der Dopplerwinkel der einzelnen Piezokeramiken berechnet. Die Gefäßtiefe und der Gefäßdurchmesser ergeben sich aus der Torvariation unter Berücksichtigung von Schallgeschwindigkeit und Einfallswinkel zur Normalen. Der Dopplerwinkel ergibt sich aus den mittleren Frequenzverschiebungen für zwei Piezokeramiken wie folgt:

| Parameter | Zeichen |
|---|---|
| Volumenfluss | $\dot{V}$ |
| Mittlere Geschwindigkeit im Gefäß | $\bar{v}$ |
| Gefäßdurchmesser | d |
| Ultraschall-Sendefrequenz | $f_0$ |
| Schallgeschwindigkeit | c |
| Doppler-Sendewinkel | $\alpha_{SE}$ |
| Doppler-Empfangswinkel | $\alpha_{EM}$ |
| Mittlere Frequenzverschiebung | $\overline{\Delta f}$ |
| Winkel einer Piezokeramik zur Schallaustrittsfläche | $\beta$ |

Volumenfluss in Rohren:

$$\dot{V} = \bar{v} \; \frac{\pi}{4} \, d^2 \quad (1)$$

Dopplergesetz:

$$\overline{\Delta}\mathrm{f} = \mathrm{f}_0 \; \frac{\bar{\mathrm{v}}}{\mathrm{c}} \; (\cos \alpha_{\mathrm{SE}} + \cos \alpha_{\mathrm{EM}}) \quad (2)$$

Für zwei Empfänger bei Messung mit gleichem Sender zur gleichen Zeit gilt:

$$\overline{\Delta f_1} = f_0 \, \frac{\bar{v}}{c} \, (\cos \alpha_{SE} + \cos \alpha_{EM1}) \qquad (3)$$

$$\overline{\Delta f_2} = f_0 \, \frac{\bar{v}}{c} \, (\cos \alpha_{SE} + \cos \alpha_{EM2}) \qquad (4)$$

Über die bekannten Winkel ($\beta_1$, $\beta_2$) aus der Konstruktion und Berücksichtigung des Brechungsgesetzes lassen sich alle Winkel in Abhängigkeit von $\alpha_{EM1}$ darstellen:

$$\alpha_{SE} = \alpha_{EM1} + \beta'_1 \qquad \alpha_{EM2} = \alpha_{EM1} + \beta'_2 \qquad (5)$$

Aus Division von (3) und (4) und Einsetzen von (5) ergibt sich eine Gleichung aus der man den Dopplerwinkel $\alpha_{EM1}$ bestimmen kann:

$$\frac{\overline{\Delta f_1}}{\overline{\Delta f_2}} = \frac{(\cos(\alpha_{EM1} + \beta_1) + \cos \alpha_{EM1})}{\cos(\alpha_{EM1} + \beta_1) + \cos(\alpha_{EM1} + \beta_2)} \qquad (6)$$

Erfindungsgemäß wird eine Messung mit dem Modus 2 durchgeführt. Im Modus 2 arbeitet eine Piezokeramik als Sender, während alle verfügbaren Piezokeramiken als Empfänger arbeiten. Die reflektierten Ultraschallsignale werden von den Empfängern zeitgleich aufgenommen. Das alle verfügbaren Piezokeramiken als Empfänger arbeiten, bedeutet, dass im cw-Betrieb eine Piezokeramik sendet während alle übrigen Piezokeramiken empfangen und im Puls-Doppler Betrieb alle Piezokeramiken inklusive der Piezokeramik, die als Sender agiert, empfangen. Die Synchronisierung der Messung findet über systeminterne Trigger statt.

Erfindungsgemäß wird ein aus Modus 1 ermittelter und in der Messelektronik abgespeicherter Parametersatz abgerufen und für die Konfiguration aller Piezokeramiken genutzt.

Hierbei kann ein Parametersatz durch einen Anwender ausgewählt werden oder aber es wird automatisch der Parametersatz ausgewählt, der das beste Signal zu Rauschverhältnis (SNR) bei der Messung im Modus 1 aufgewiesen hat. Bevorzugt wird der Parametersatz automatisch ausgewählt. Die Konfiguration bestimmt, welche Piezokeramik Ultraschallsignale sendet und wie tief und wie groß das Empfangstor jeder Piezokeramik ist. Erfindungsgemäß findet im Modus 2 eine kontinuierliche Puls-Doppler-Messung mit gleichbleibenden Einstellungen statt. Anhand der empfangenen Daten werden Spektrogramme errechnet. Bei der Berechnung können hinterlegte Informationen zum Dopplerwinkel einer jeden Piezokeramik genutzt werden, um aus den Frequenzwerten der Spektrogramme Flussgeschwindigkeiten zu ermitteln. Aus den errechneten Daten werden einhüllende Funktionen bzw. Flusskurven errechnet. Aus den Flusskurven können dann mehrere Flussinformationen wie zum Beispiel mittlere Blutflussgeschwindigkeit, systolische Spitzengeschwindigkeit, end-diastolische Spitzengeschwindigkeit, Puls / Reanimationsfrequenz ermittelt werden.

Erfindungsgemäß wird damit die Geschwindigkeit des Blutflusses und/oder der Volumenfluss aus den Messignalen der Messung mit Modus 2 und der berechneten Gefäßtiefe, des Gefäßdurchmessers und der Dopplerwinkel der Piezokeramiken errechnet.

In einer Ausführungsform wird der berechnete Blutfluss über eine Anzeige an der Ultraschallvorrichtung dargestellt.

In einer Ausführungsform kann der ermittelte Blutflusswerte entlang der Zeit ausgewertet werden, um somit zu bestimmen, ob die Ultraschallvorrichtung gut über dem Blutgefäß positioniert ist und ein Blutfluss entsteht, der zuvor einprogrammierten Sollwerten entspricht. Hierfür wird in der Messelektronik mindestens ein Sollwert für den Blutfluss hinterlegt. LEDs, welche sich entweder an der Ultraschallvorrichtung befinden, zeigen dem Nutzer das Ausmaß des erzeugten Blutflusses und/oder die Güte des ermittelten Signals und/oder den Blutflussverlauf der letzten Minute(n) an. Die Anzeigen können in einer Ausführungsform über eine LED-Leiste für die momentane Geschwindigkeit, eine Status-LED für den Flussverlauf (z.B. jeweils rot-gelb-grün für 1 Minute, 10 Minuten, 1/2h) und Status-LEDs (z.B. rot-gelb-grün) für die Signal-/Lagegüte realisiert werden.

In einer weiteren Ausführungsform wird ein akustisches Signal ausgegeben, wenn der berechnete Blutfluss von einem in der Messelektronik hinterlegten Sollwert abweicht.

Die Ultraschallvorrichtung und das erfindungsgemäße Verfahren bieten zahlreiche Vorteile gegenüber dem Stand der Technik.

- Es wird ein Doppler-Messgerät zur Verfügung gestellt, welches flexibel und schnell am Patienten angebracht werden kann, ohne dass das Gerät von einem Nutzer festgehalten werden muss oder die Position nach der Befestigung nicht geändert werden kann. Dies ist mit den aus dem Stand der Technik bekannten Geräten bisher nicht möglich.
- Weiterhin etabliert die Erfindung gepulste dopplersonographische Messungen beim Reanimationsszenario und die damit verbundenen Vorteile des gesteuerten Sendens und Empfangens von Ultraschallwellen aus unterschiedlichen Winkeln. Durch diesen Ansatz kann das unter der Ultraschallvorrichtung liegende Blutgefäß und die darin stattfindenden Bewegungen gezielter auf seine hämodynamischen Eigenschaften untersucht werden.

- Eine geräteinterne Auswertung und Berechnung von Blutflussparametern erlaubt zudem die sofortige Anzeige der relevanten Messparameter über verbaute LEDs und liefert damit Echtzeit-Feedback.
- Die Erfindung verzichtet auf selbstklebende Materialien zur Befestigung der Ultraschallvorrichtung am Hals des Patienten. Stattdessen werden Halterungen aus Kunststoff genutzt, welche an einer Laerdal-Stiffneck Halskrause befestigt werden können oder über ein dehnbares Textil oder eine eigens entwickelte Halskrause am Hals angebracht werden können. Mithilfe von manuell aufblasbaren Luftkissen kann die Position und der Anpressdruck am Hals je nach Patient angepasst werden. Eine Betätigung eines Rückschlagventils der Pumpe und die Entriegelung eines Schlussmechanismus der Halskrause garantieren eine unkomplizierte Abnahme des Messmoduls. Die Position der Ultraschallvorrichtung wird durch Verschieben der Halterung an der Halskrause geändert, wobei aufgrund des Anpressdrucks die Position der Ultraschallvorrichtung bei geschlossener Halskrause nicht veränderlich ist.
- Die benutzte Sonde für die Ultraschallmessung besteht aus mindestens drei Piezokeramiken, welche unterschiedliche Einfallwinkel für die dopplersonographische Messung besitzen. Bei einer aufeinander abgestimmten Puls-Doppler-Messung ist es möglich, dass eine Piezokeramik als Sender und Empfänger fungiert. Aus den Messwerten kann die Position des Blutgefäßes in Abhängigkeit der Pulsreflektionen und relativen Magnitude der Dopplersignale bestimmt werden.
- Da der Dopplerwinkel direkt in die Berechnung der Flussgeschwindigkeiten eingeht, lassen sich diese genau bestimmen. Die Winkelpositionen der Piezokeramiken sind fix und der Dopplerwinkel jeder Piezokeramik ist aus den Messungen genau bekannt. An herkömmlichen Geräten muss der Dopplerwinkel vom Nutzer manuell eingezeichnet werden, was zu Ungenauigkeiten führt. Daher bietet die automatisierte Ermittlung des Dopplerwinkels einen Vorteil gegenüber der herkömmlichen Methode bei der Bestimmung der Flussgeschwindigkeiten. Eine Ermittlung des Gefäßdurchmessers zusammen mit der Bestimmung der Flussgeschwindigkeiten ermöglicht die Quantifizierung des Volumenstroms der Erythrozyten.
- Wird eine Herzdruckmassage durchgeführt, besteht die Möglichkeit die Frequenzverläufe entlang der Zeit auf Gefäßbewegungen zu untersuchen und daran festzustellen, welche Piezokeramik besser für die Blutflussberechnung geeignet ist und welche patientenspezifisches Empfangstor angewendet werden sollte. Die Informationen bezüglich der Gefäßbewegung sind im niederfrequenten Bereich des Spektrums enthalten und leistungsstärker als Frequenzunterschiede, die durch den Blutfluss entstehen. Durch Vergleichswerte der Leistung in den Frequenzbereichen aus zuvor durchgeführten Untersuchungen kann somit bestimmt werden, ob die Ultraschallvorrichtung das Blutgefäß ausreichend erfasst oder eine Verschiebung der Position der Ultraschallvorrichtung oder eine Anpassung des Doppler-Empfangstors angeraten ist. So besteht die Möglichkeit durch die Messelektronik eine iterative Verschiebung des Empfangstors durchzuführen. Eine Auswertung der erreichten Signalstärken kann Aufschluss über die Tiefe des Gefäßes geben und ob die Mitte des Gefäßes getroffen wird.
- Die Erfindung bietet zudem die Möglichkeit auf die Anbindungen zu Drittgeräten wie Monitore o.ä. als Anzeige zu verzichten. Anstatt der Datenübertragung per Bluetooth oder Wireless-LAN werden Messwerte sofort ausgewertet und für die Anzeige des Reanimationsverlaufs genutzt. Die übersichtliche Anzeige liefert dem Nutzer ein einfaches und schnelles Feedback, welches die Erfassung des Blutgefäßes und/oder den momentanen Fluss und/oder die kürzlich erfassten Blutflusswerte umfasst. Zu diesem Zweck können LEDs genutzt werden, die den approximierten, momentanen Blutflusswert anzeigen und/oder die Signalstärke des Messsignals und/oder die Bewertung des approximierten Flusses entlang der vergangenen Minute(n).

Der messtechnische Aufbau der erfindungsgemäßen Vorrichtung bietet darüber hinaus die folgenden Vorteile:

- ein Vergleich von Puls-Doppler-Daten unterschiedlich angewinkelter Piezokeramiken mit großem Sample-Volumen ermöglicht einen Aufschluss über die prinzipielle Abdeckung eines blutdurchflossenen Gefäßes durch die Schallfelder der Piezokeramiken;

- ein Vergleich von Puls-Doppler-Daten unterschiedlich angewinkelter Piezokeramiken mit kleinen Sample-Volumen und unterschiedlichen Tortiefen ermöglicht die Berechnungen der Gefäßtiefe, des Gefäßdurchmessers und der Piezokeramik spezifischen Dopplerwinkel zum Gefäß ohne a-priori Wissen;

- eine automatische Intensitätsbewertung der Spektrogramme und Flusskurvenberechnung ermöglicht eine optimale Messkonfigurationen der Piezokeramiken ohne manuelle Eingabe;

- die Anpassung der Tortiefe auf die patientenspezifische Tortiefe verbessert das Signal Rausch Verhältnis maßgeblich;

- eine Substitution der Piezokeramik-spezifischen Dopplerwinkel bei der Berechnung der Doppler-Spektrogramme ermöglicht eine automatisch quantifizierte Geschwindigkeitsberechnung innerhalb des erfassten Sample-Volumen;
- die Substitution der Piezokeramik-spezifischen Dopplerwinkel umgeht die gebräuchliche Verwendung des Winkels zwischen Piezokeramik und Gefäßachse zur Flussberechnung und ermöglicht dadurch eine robustere Berechnung der Flussgeschwindigkeiten;
- die Approximation des Gefäßdurchmessers und die Quantifizierung der reellen Blutflussgeschwindigkeiten ermöglicht die Quantifizierung des Volumenflusses;
- ein Vergleich der Spektrogramm-Intensitäten, der ermittelten Flussinformationen und des Frequenzverhaltens der errechneten Flusskurven mit abgespeicherten Richtwerten ermöglicht eine Beurteilung der Sensorpositionierung und des Blutflusses;
- ohne a-priori Wissen kann das System zur Darstellung des momentan erfassbaren Blutflusses, der Qualität der Flusssignale und der Qualität des Blutflusses über einen vergangenen Zeitraum in einer einfachen technischen Ausführung mithilfe mehrfarbiger LEDs genutzt werden.

Neben der Messung des Blutflusses währen einer Reanimation kann die erfindungsgemäße Ultraschallvorrichtung und das erfindungsgemäße Verfahren auch genutzt werden zur:

- nicht-invasive Herz-Zeit-Volumen-Approximation oder Herz-Puls-Analysen auf der Intensivstation
- nicht-invasive Untersuchung von Anzeichen peripherer Verschlusskrankheiten.

Im Folgenden wird die vorliegende Erfindung anhand von 7 Figuren und 3 Ausführungsbeispielen näher erläutert.

Figur 1 stellt eine Ultraschallvorrichtung dar;

Figur 2 stellt eine Ultraschallvorrichtung an einem Blutgefäß in einem Körpergewebe dar;

Figur 3 stellt eine Laerdal-Stiffneck Halskrause mit einer Ultraschallvorrichtung dar;

Figur 4 stellt einen Ausschnitt einer Laerdal-Stiffneck Halskrause mit einer Ultraschallvorrichtung dar;

Figur 5 stellt einen Ausschnitt einer Laerdal-Stiffneck Halskrause mit einer Ultraschallvorrichtung dar;

Figur 6 stellt symbolisch die Einhüllende der Intensitäten eines Spektrogramms und die Signale, die durch Gefäßbewegungen verursacht werden;

Figur 7 (A) bis (E) stellen Einhüllende der Intensitäten von Spektrogrammen dar.

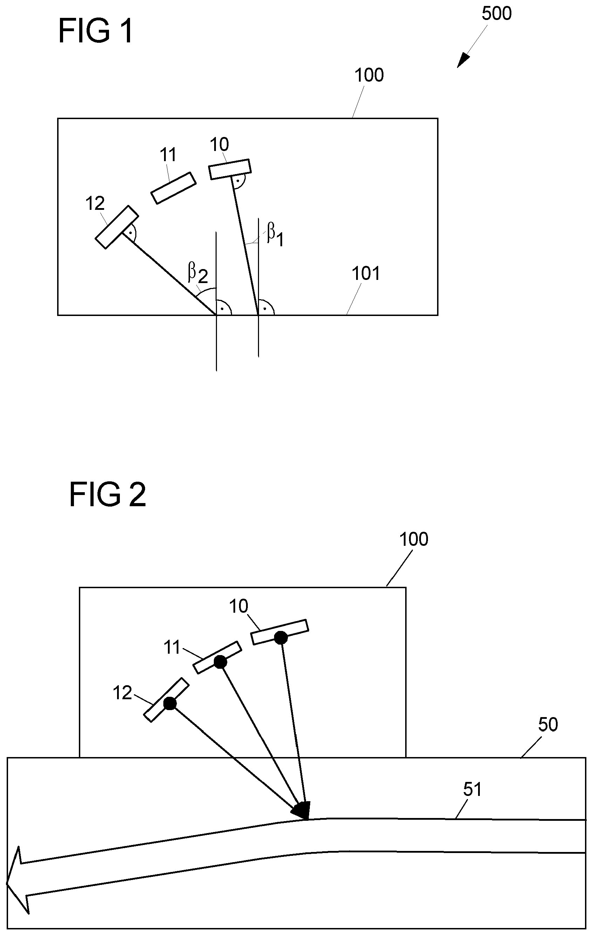

**Figur 1** stellt in vereinfachter weise eine Ultraschallvorrichtung 500 dar. Die Ultraschallvorrichtung 500 weist drei Piezokeramiken 10, 11, 12 auf. Die Piezokeramik 10 ist im Winkel $\beta_1$ und die Piezokeramik 12 im Winkel $\beta_2$ im Gehäuse 100 angeordnet. Der Winkel ergibt sich als Winkel zwischen der Normalen der Piezokeramik und der Normalen der Schallaustrittsfläche 101 des Gehäuses, durch die der Ultraschall ausgestrahlt wird. Der Übersichtlichkeit halber ist der Winkel für die Piezokeramik 11 nicht gekennzeichnet.

**Figur 2** stellt eine Ultraschallvorrichtung 500 an einem Blutgefäß 51 in einem Körpergewebe 50 dar. Die drei Piezokeramiken 10, 11, 12 sind jeweils in unterschiedlichen Winkeln im Gehäuse 100 angeordnet und strahlen den Ultraschall daher entsprechend in unterschiedlichen Winkeln in das Blutgefäß 51, welches im Körpergewebe 50 liegt ein.

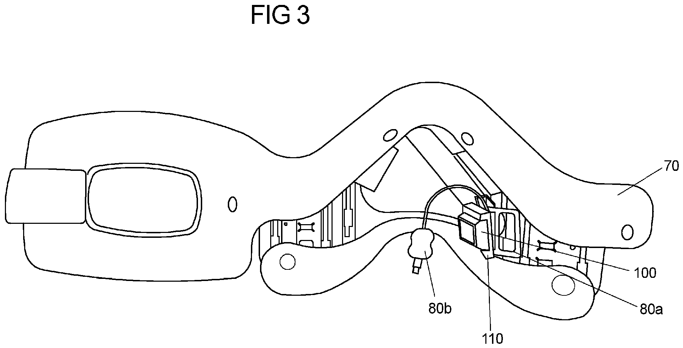

**Figur 3** stellt eine Laerdal-Stiffneck Halskrause 70 dar. An dieser ist eine Ultraschallvorrichtung 500 angebracht. Die Ultraschallvorrichtung 500 weist ein Gehäuse 100 auf, in dem drei Piezokeramiken 10, 11, 12 angeordnet sind. Das Gehäuse 100 weist weiterhin eine Halterung 1010 auf, mit der das Gehäuse an der Laerdal-Stiffneck Halskrause 70 befestigt ist. Weiterhin weit die Ultraschallvorrichtung 500 ein Luftkissen 80a auf, dessen Luftfüllung über den Zugang 80b reguliert werden kann. Dies kann beispielsweise über eine Spritze erfolgen. In dieser Ausführungsform ist die Messelektronik in einem weiteren Gehäuse untergebracht, welches an der vom Patienten abgewandten Seite der Laerdal-Stiffneck Halskrause 70 angebracht ist. Das Gehäuse 100 mit den Piezokeramiken 10, 11, 12 ist durch ein Kabel mit dem weiteren Gehäuse, in dem sich die Messelektronik befindet verbunden.

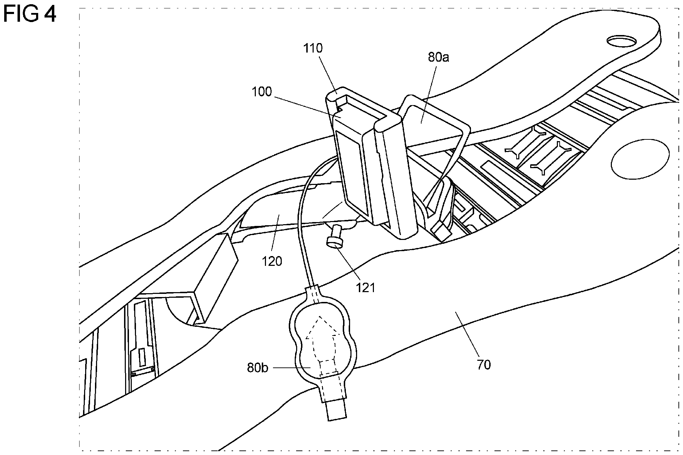

**Figur 4** stellt einen Ausschnitt einer Laerdal-Stiffneck Halskrause 70 mit einer Ultraschallvorrichtung 500 dar. Die zur Figur 3 beschriebenen Merkmale sind noch einmal deutlicher zu erkennen. Die Beschreibung der Figur 3 trifft in gleicher Wiese auf die Figur 4 zu.

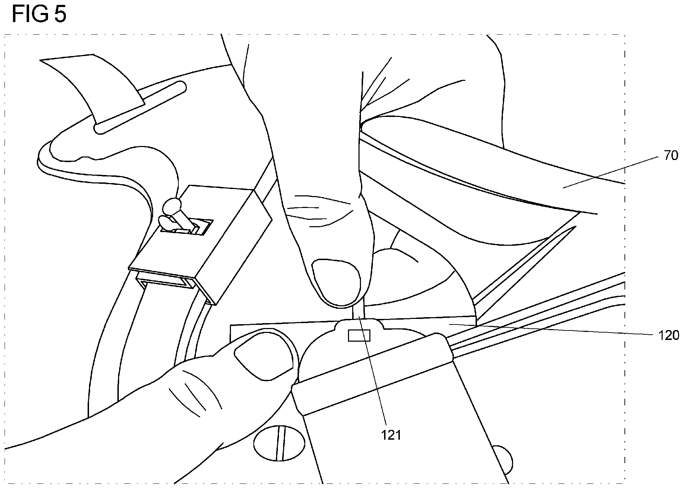

**Figur 5** stellt einen Ausschnitt einer Laerdal-Stiffneck Halskrause 70 mit einer Ultraschallvorrichtung 500 dar. Es ist die Rückseite der Ultraschallvorrichtung 500 zu erkennen. Die Halterung der Ultraschallvorrichtung 500 ist in dieser Ausführungsform so gestaltet, dass die Position des Gehäuses 100 und damit der Piezokeramiken 10, 11, 12 an Laerdal-Stiffneck Halskrause 70 verändert werden kann. Hierdurch kann die Position des Gehäuses 100 am Hals eines Patienten justiert werden. Hierfür wird die Position des Gehäuse 100 über die Stellschraube 121 und den Positionierstreifen 120 verändert. Durch Drehen der Stellschraube kann das Gehäuse entlang des Positionierstreifens bewegt werden.

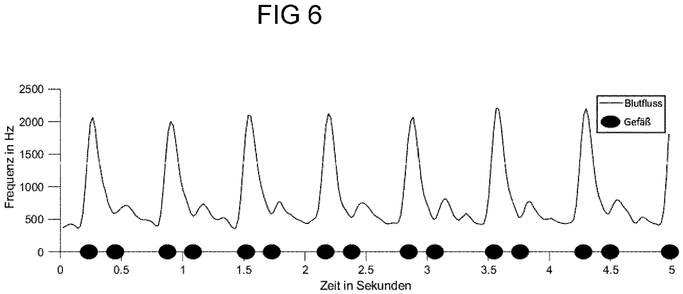

**Figur 6** stellt symbolisch die Einhüllende der Intensitäten eines Spektrogramms dar. Die Einhüllende spiegelt dabei den Signalanteil wider, der durch den Blutfluss im Gefäß verursacht wird. Die Punkte auf der Zeitachse symbolisieren die Signale, die durch die Gefäßbewegung hervorgerufen werden. Wobei der erste Punkt jeweils das Signal widerspiegelt, welches durch die Gefäßbewegung beim Aufdehnen des Gefäßes verursacht wird und der unmittelbar darauffolgende Punkt das Signal, welches durch die Gefäßbewegung beim Zusammenziehen des Gefäßes verursacht wird.

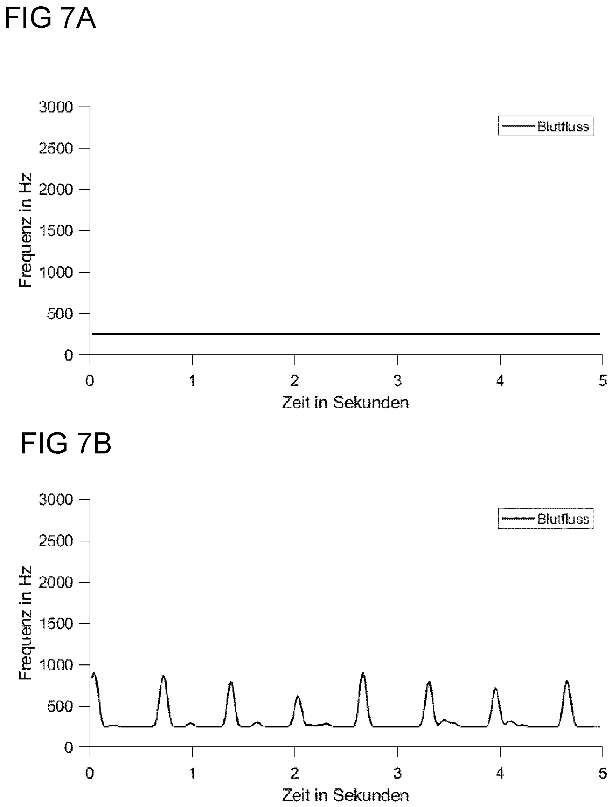

**Figur 7 (A) bis (E)** stellen Einhüllende der Intensitäten von Spektrogrammen dar, die mit einer Piezokeramik für verschiedene Tortiefen aufgenommen wurden. Die Figuren sind im Ausführungsbeispiel 2 näher beschrieben.

## Ausführungsbeispiel 1

Die Ultraschallvorrichtung wurde in einen Laerdal Stiffneck integriert. In dieser Form kann es nach der sicheren Lagerung eines Patienten ohne Spontankreislauf am Patienten angebracht werden. Das Messmodul befand sich dabei an einer Stelle am Stiffneck, der bei mehreren Probanden optimal für die Erfassung des Blutflusses in der Carotis anhand von Dopplersonographie war. Nach der Anbringung wurde die Flächenabdeckung des Ultraschallsensors überprüft, wobei die Ultraschallsensorabdeckung des Hales bzw. der Anpressdruck durch das Aufblasen des Luftkissens optimiert wurden.

## Ausführungsbeispiel 2

Die Ultraschallvorrichtung wurde in einen Laerdal Stiffneck integriert und an einem Phantom mit einem simulierten Blutfluss angebracht. Als Phantom wurde ein Phantom aus Agar-Agar-Gel mit Aluminiumoxid-Zugabe verwendet. Das Phantom leitete damit Ultraschall entsprechend den in der DIN IEC 60601-2-37 gestellten Anforderungen an Gewebephantome zur Prüfung von Ultraschallgeräten.

Eine Pumpe zur Erzeugung eines simulierten Blutflusses war über elastische Silikonschläuche und Tüllen in einem geschlossenen Kreislauf mit dem Phantom verbunden. Ein Silikonschlauch simulierte damit ein Blutgefäß.

Die Messung wurde mit einer Ultraschallvorrichtung durchgeführt. In den Figuren 7 (A) bis (C) sind Messkurven für Messungen mit einer Piezokeramik als Sender und Empfänger für verschiedene Tortiefen dargestellt. Die Torlänge betrug jeweils 2 µs.

In der Figur 7 (A) wurde eine Tortiefe von 14 µs verwendet. In diesem Bereich konnte kein Messignal aufgenommen werden, welches durch den simulierten Blutfluss im Gefäß verursacht wurde. Die Tortiefe war nicht groß genug, so dass das Blutgefäß nicht vom Ultraschallsignal detektiert werden konnte.

In der Figur 7 (B) wurde eine Tortiefe von 18 µs verwendet. Aus dem Spektrogramm konnten erste Informationen über einen Blutfluss gewonnen werden.

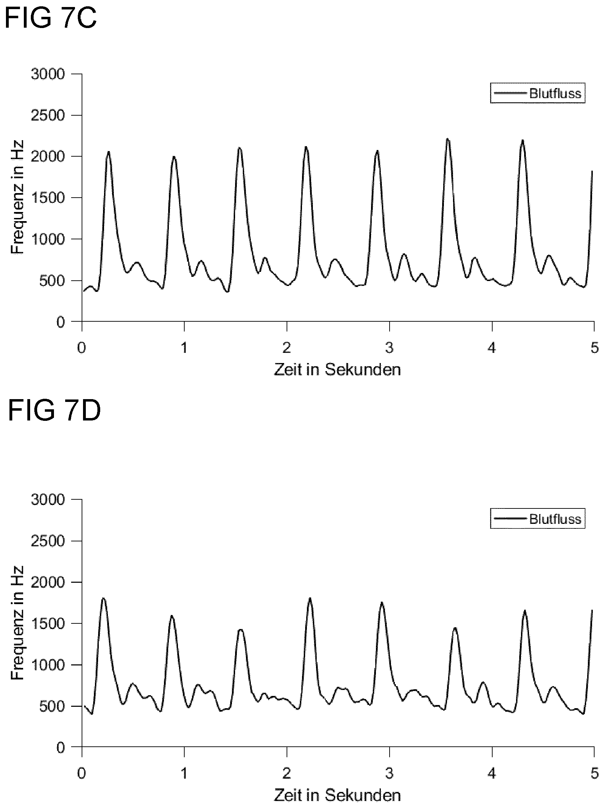

Die Tortiefe wurde daraufhin auf 22 µs verändert. Bei diesen Parametern konnte der simulierte Blutfluss im Gefäß gut abgebildet werden (Figur 7 (C)).

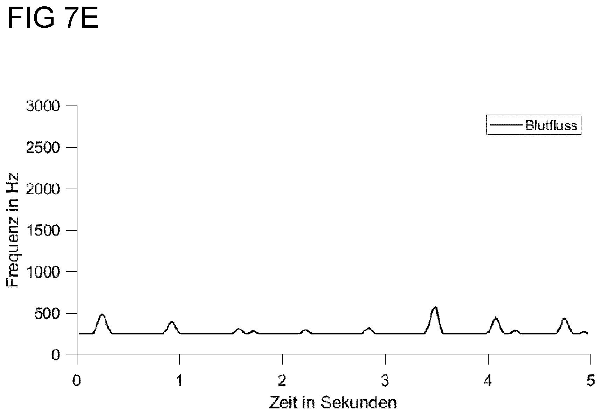

Bei noch größerer Tortiefe von 26 µs (Figur 7 (D)) konnten ebenfalls Informationen über den Blutfluss gewonnen werden, jedoch war die Intensität des Signals geringer im Vergleich zu der Messung, die in Figur 7 (C) dargestellt ist. Die Mitte des simulierten Blutstromes war bereits überschritten. Bei einer weiteren Vergrößerung der Tortiefe auf 30 µs (Figur 7 (E)) nimmt die Intensität weiter ab und es war keine Information aus dem Spektrogramm entnehmbar, mit der eine ausreichende Information über den simulierten Blutstrom gewonnen werden kann. Das Blutgefäß wurde bei dieser Tortiefe nicht mehr ausreichend abgedeckt.

Aus den Messdaten konnten folgende Informationen gewonnen werden:

Länge der Strecke für Piezokeramik bis zum Gefäßbeginn (Lage des Gefäßes)

$$18\ \mu s * 1{,}5\frac{mm}{\mu s} * \frac{1}{2} = 13{,}5\ mm$$

Länge d. abgedeckten Weges für Piezokeramik innerhalb des Gefäßes (ohne Winkelkorrektur) (Durchmesser des Gefäßes)

$$(32\ \mu s - 18\ \mu s) * 1{,}5 \frac{mm}{\mu s} * \frac{1}{2} = 10{,}5\ mm$$

Durch die Messung mit mindestens einer weiteren Piezokeramik kann gemäß den Formeln (3) bis (6) der Dopplerwinkel berechnet werden.

**Ausführungsbeispiel 3**

Es wurde eine Ultraschallvorrichtung mit drei Piezokeramiken gebaut. Die Piezokeramiken waren in einem Winkel von 0°, 10° und 25° angeordnet. Am Gehäuse der Ultraschallvorrichtung befand sich zudem eine LED-Anzeige und eine flexible textile Halterung zur Fixierung der Vorrichtung am Hals eines Patienten.

**Literatur**

[1] S.H. Friess et al. Hemodynamic directed cardiopulmonary resuscitation improves short-term survival from ventricular fibrillation cardiac arrest. Crit Care Med 2013; 41:2698-704.
[2] A. Schneider et al. 'Cerebral Resuscitation After Cardiocirculatory Arrest', Anesthesia & Analgesia, vol. 108, no. 3, pp. 971-979, Mar. 2009.
[3] M. Koch et al. Carotid Artery Ultrasound in the (peri-) Arrest Setting-A Prospective Pilot Study. J Clin Med. 2022;11(2):469. Published 2022 Jan 17. doi:10.3390/jcm11020469

**Bezugszeichenliste**

| | |
|---|---|
| 10, 11, 12 | Piezokeramik |
| 50 | Körpergewebe |
| 51 | Blutgefäß |
| 70 | Laerdal Stiffneck Halskrause |
| 80a | Luftkissen |
| 80b | Zugang |
| 100 | Gehäuse |
| 101 | Schallaustrittsfläche |
| 120 | Positionierstreifen |
| 121 | Stellschraube |
| 500 | Ultraschallvorrichtung |

**Patentansprüche**

**1.** Nichtinvasives Verfahren zur Messung des Blutstromes in einem Blutgefäß mit einer Ultraschallvorrichtung (500) aufweisend eine Messelektronik, mindestens drei Piezokeramiken (10, 11, 12) und ein Gehäuse (100), wobei

jede Piezokeramik (10, 11, 12) in einem anderen Winkel zur Schallaustrittsfläche des Gehäuses (101) im Gehäuse (100) angeordnet ist;
das Gehäuse (100) weiterhin mindestens ein Mittel zum Befestigen des Gehäuses an einem Patienten oder an einem Hilfsmittel aufweist;
**dadurch gekennzeichnet, dass** das Verfahren die Schritte aufweist

a) Ausführen einer Messung mit einem Modus 0, in dem eine Piezokeramik (10, 11, 12) als Sender arbeitet, während alle verfügbaren Piezokeramiken (10, 11, 12) als Empfänger arbeiten;
b) Beurteilung ob bei der Messung ein blutdurchströmtes Blutgefäß erfasst wird anhand der Messwerte mit dem Modus 0 und Ausgabe einer Information, ob bei der Messung ein blutdurchströmtes Blutgefäß erfasst wird;
c) Ausführen einer Messung mit einem Modus 1, indem nacheinander jede Piezokeramik (10, 11, 12) als Sender arbeitet, während alle verfügbaren Piezokeramiken (10, 11, 12) als Empfänger arbeiten, wobei die reflektierten Ultraschallsignale von den Empfängern zeitgleich aufgenommen werden und wobei für jede Piezokeramik die Messtortiefe variiert wird;
d) Berechnung der Gefäßtiefe, des Gefäßdurchmessers und der Dopplerwinkel der einzelnen Piezokeramiken (10, 11, 12) aus den Messdaten der Messung im Modus 1;
e) Ausführen einer Messung mit einem Modus 2, indem eine Piezokeramik (10, 11, 12) als Sender arbeitet, während alle verfügbaren Piezokeramiken (10, 11, 12) als Empfänger arbeiten, wobei die reflektierten Ultraschallsignale von den Empfängern zeitgleich aufgenommen werden;
f) Berechnung der Geschwindigkeit des Blutflusses aus den Messsignalen der Messung mit Modus 2 und der berechneten Gefäßtiefe, des Gefäßdurchmessers und der Dopplerwinkel der Piezokeramiken (10, 11, 12).

**2.** Verfahren gemäß Anspruch 1, **dadurch gekennzeichnet, dass** das Verfahren weiterhin den Schritt aufweist, dass der berechnete Blutfluss über eine Anzeige am Gehäuse (100) dargestellt wird.

**3.** Verfahren gemäß einem der Ansprüche 1 oder 2, **dadurch gekennzeichnet, dass** das Verfahren weiterhin den Schritt aufweist, dass ein akustisches Signal ausgegeben wird, wenn der berechnete Blutfluss von einem in der Messelektronik hinterlegten Sollwert abweicht.

## Claims

**1.** Non-invasive method for measuring blood flow in a blood vessel by means of an ultrasound device (500) comprising measuring electronics, at least three piezoceramic elements (10, 11, 12) and a housing (100),

each piezoceramic element (10, 11, 12) being arranged in the housing (100) at a different angle to the sound emission surface of the housing (101);
the housing (100) further having at least one means for attaching the housing to a patient or to an auxiliary means;
**characterized in that** the method comprises the steps of

a) performing a measurement using a mode 0 in which one piezoceramic element (10, 11, 12) acts as a transmitter, while all available piezoceramic elements (10, 11, 12) act as receivers;
b) assessing whether a blood-filled blood vessel is detected during the measurement on the basis of the measured values using the mode 0, and outputting information as to whether a blood-filled blood vessel is detected during the measurement;
c) performing a measurement using a mode 1, in which each piezoceramic element (10, 11, 12) acts as a transmitter in succession, while all available piezoceramic elements (10, 11, 12) act as receivers, the reflected ultrasound signals being received simultaneously by the receivers and the measurement gate depth being varied for each piezoceramic element;
d) calculating the vessel depth, vessel diameter and Doppler angles of the individual piezoceramic elements (10, 11, 12) from the measurement data from the measurement using the mode 1;
e) performing a measurement using a mode 2, in which one piezoceramic element (10, 11, 12) acts as a transmitter, while all available piezoceramic elements (10, 11, 12) act as receivers, the reflected ultrasound signals being received simultaneously by the receivers;
f) calculating the speed of blood flow from the measurement signals from the measurement using the mode 2 and the calculated vessel depth, vessel diameter and Doppler angles of the piezoceramic elements (10, 11, 12).

**2.** Method according to claim 1, **characterized in that** the method further comprises the step of displaying the calculated blood flow by means of a display on the housing (100).

**3.** Method according to claim 1 or claim 2, **characterized in that** the method further comprises the step of emitting an acoustic signal if the calculated blood flow deviates from a setpoint value stored in the measuring electronics.

## Revendications

1. Procédé non invasif pour la mesure du flux sanguin dans un vaisseau sanguin avec un dispositif à ultrasons (500) présentant une électronique de mesure, au moins trois céramiques piézoélectriques (10, 11, 12) et un boîtier (100), dans lequel

   chaque céramique piézoélectrique (10, 11, 12) est disposée dans le boîtier (100) selon un angle différent par rapport à la surface de sortie du son du boîtier (101) ;
   le boîtier (100) présente en outre au moins un moyen pour la fixation du boîtier à un patient ou à un moyen auxiliaire ;
   **caractérisé en ce que** le procédé présente les étapes consistant à :

   a) effectuer une mesure avec un mode 0, dans lequel une céramique piézoélectrique (10, 11, 12) fonctionne comme émetteur, tandis que toutes les céramiques piézoélectriques (10, 11, 12) disponibles fonctionnent comme récepteur ;
   b) évaluer si un vaisseau sanguin traversé par le sang est détecté lors de la mesure à l'aide des valeurs de mesure avec le mode 0 et sortir des informations si un vaisseau sanguin traversé par le sang est détecté lors de la mesure ;
   c) effectuer une mesure avec un mode 1 en faisant fonctionner successivement chaque céramique piézoélectrique (10, 11, 12) comme émetteur, tandis que toutes les céramiques piézoélectriques (10, 11, 12) disponibles fonctionnent comme récepteur, dans lequel les signaux ultrasonores réfléchis sont reçus simultanément par les récepteurs et dans lequel la profondeur de porte de mesure est modifiée pour chaque céramique piézoélectrique ;
   d) calculer la profondeur de vaisseau, le diamètre de vaisseau et l'angle Doppler des différentes céramiques piézoélectriques (10, 11, 12) à partir des données de mesure de la mesure dans le mode 1 ;
   e) effectuer une mesure avec un mode 2 en faisant fonctionner une céramique piézoélectrique (10, 11, 12) comme émetteur, tandis que toutes les céramiques piézoélectriques (10, 11, 12) disponibles fonctionnent comme récepteur, dans lequel les signaux ultrasonores réfléchis sont reçus simultanément par les récepteurs ;
   f) calculer la vitesse du flux sanguin à partir des signaux de mesure de la mesure avec le mode 2 et de la profondeur de vaisseau calculée, du diamètre de vaisseau et de l'angle Doppler des céramiques piézoélectriques (10, 11, 12).

2. Procédé selon la revendication 1, **caractérisé en ce que** le procédé présente en outre l'étape consistant à représenter le flux sanguin calculé par l'intermédiaire d'un moyen d'affichage sur le boîtier (100).

3. Procédé selon l'une des revendications 1 ou 2, **caractérisé en ce que** le procédé présente en outre l'étape consistant à émettre un signal acoustique lorsque le flux sanguin calculé s'écarte d'une valeur de consigne enregistrée dans l'électronique de mesure.

FIG 1

500
100
11
10
12
$\beta_1$
$\beta_2$
101

FIG 2

100
10
11
12
50
51

FIG 3

70
100
80a
80b
110

FIG 4

110
80a
100
120
121
80b
70

FIG 5

70
120
121

FIG 6

2500
2000
1500
1000
500
0
Frequenz in Hz
Blutfluss
Gefäß
0
0.5
1
1.5
2
2.5
3
3.5
4
4.5
5
Zeit in Sekunden

FIG 7A

Blutfluss
Frequenz in Hz
3000
2500
2000
1500
1000
500
0
0
1
2
3
4
5
Zeit in Sekunden

FIG 7B

Blutfluss
Frequenz in Hz
3000
2500
2000
1500
1000
500
0
0
1
2
3
4
5
Zeit in Sekunden

FIG 7C

Blutfluss
Frequenz in Hz
3000
2500
2000
1500
1000
500
0
0
1
2
3
4
5
Zeit in Sekunden

FIG 7D

Blutfluss
Frequenz in Hz
3000
2500
2000
1500
1000
500
0
0
1
2
3
4
5
Zeit in Sekunden

FIG 7E

3000
2500
2000
1500
1000
500
0
Frequenz in Hz
Blutfluss
0
1
2
3
4
5
Zeit in Sekunden

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 3823536 A, Joseph Eibl
- WO 2017096487 A
- EP 3505071 A1
- US 2006241460 A1
- US 2017332995 A1


### In der Beschreibung aufgeführte Nicht-Patentliteratur


- **S.H. FRIESS et al.** Hemodynamic directed cardiopulmonary resuscitation improves short-term survival from ventricular fibrillation cardiac arrest. *Crit Care Med*, 2013, vol. 41, 2698-704
- **A. SCHNEIDER et al.** Cerebral Resuscitation After Cardiocirculatory Arrest. *Anesthesia & Analgesia*, March 2009, vol. 108 (3), 971-979
- **M. KOCH et al.** Carotid Artery Ultrasound in the (peri-) Arrest Setting-A Prospective Pilot Study. *J Clin Med.*, 2022, vol. 11 (2), 469